# EUROPEAN PATENT APPLICATION

(11) **EP 3 733 202 A1**
(43) Date of publication of application: **04.11.2020**
(21) Application number: 18893813.8
(22) Date of filing: 28.12.2018
(51) Int. Cl.: A61K 39/395, A61K 31/444, C07K 16/28, A61P 35/00

(54) **USE OF COMBINED TREATMENT OF PD-1 ANTIBODY AND APATINIB FOR TREATING TRIPLE NEGATIVE BREAST CANCER**

(30) Priority: 29.12.2017 CN 201711481358; 17.09.2018 CN 201811082708
(71) Applicant: Jiangsu Hengrui Medicine Co., Ltd., Lianyungang, Jiangsu 222047 (CN); Suzhou Suncadia Biopharmaceuticals Co., Ltd., Suzhou, Jiangsu 215126 (CN)
(72) Inventor: WANG, Quanren, Lianyungang, Jiangsu 222047 (CN); DAI, Zongfei, Lianyungang, Jiangsu 222047 (CN)
(74) Representative: Regimbeau
(86) International application number: PCT/CN2018/124563
(87) International publication number: WO 2019/129168

(57) **Abstract**

The present invention relates to a use of a combined treatment of a PD-1 antibody and Apatinib for treating Triple Negative Breast Cancer. In particular, the present invention relates to a use of an anti-PD-1 antibody in combination with Apatinib in the preparation of a drug for Triple Negative Breast Cancer.

## Description

### FIELD OF THE INVENTION

The present invention relates to use of an anti-PD-1 antibody or antigen-binding fragment thereof in combination with Apatinib or a pharmaceutically acceptable salt thereof in the manufacture of a medicament for the treatment of Triple Negative Breast Cancer.

### BACKGROUND OF THE INVENTION

Breast cancer is the most common malignant tumor that endangers women's health, has the highest incidence rate among female cancers in China and the incidence is still on rise. Breast cancer is a collection of diseases, at least can be divided into four molecular subtypes: Luminal A, luminal B, HER2-overexpressing, and triple negative. Three Negative Breast Cancer accounts for about 15% of the total breast cancer cases, and is characterized in high early relapse rate, high distant metastasis rate and poor prognosis. In absence of effective targeted therapy drugs, ABC3 currently only recommends low-toxicity chemotherapy for Triple Negative Breast Cancer. However, resistance to chemotherapy often occurs during the treatment. Therefore, it has become a hot and difficult issue to find effective targeted drugs for the treatment of Triple Negative Breast Cancer.

Apatinib, a small molecule tyrosine kinase inhibitor disclosed in WO2005000232A, highly selectively competes for the ATP-binding site with the intracellular VEGFR-2, blocks the downstream signaling, inhibits neovascularization in tumor, and finally achieves the purpose of treating tumors. The structural formula of Apatinib is shown in Formula (I).

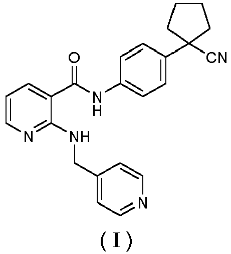

CN101676267A discloses a series of Apatinib salts, such as mesylate, hydrochloride, maleate, and the like. Preclinical animal experiments disclosed in CN101675930A also show that Apatinib in combination with cytotoxic drugs (such as oxaliplatin, 5-Fu, docetaxel, and doxorubicin) can significantly increase their efficacy.

In recent years, breakthrough progress has been achieved in tumor immunotherapy. A number of clinical studies have shown that anti-PD-1/PD-L1 antibodies show remarkable efficacy in a variety of advanced solid tumors (such as melanoma, renal cancer, non-small cell lung cancer, and breast cancer), with Overall Response Rate (ORR) of about 10%-40% against different solid tumors, and are most effective against malignant melanoma (about 36%-53%). WO2015085847A discloses a novel anti-PD-1 antibody, which is currently in clinical trials and has shown some antitumor effect. However, treatment with anti-PD-1/PD-L1 antibody alone did not achieve good efficacy for many Triple Negative Breast Cancer patients (Nanda R, Chow LQ, Dees EC et al. Pembrolizumab in Patients with Advanced Triple Negative Breast Cancer: Phase Ib KEYNOTE-012 Study. J Clin Oncol 2016; 34: 2460-2467). Therefore, it is important to find targeted drugs that can enhance the efficacy of anti-PD-1/PD-L1 antibodies when being administrated in combination with the antibodies.

### SUMMARY OF THE INVENTION

The present invention provides use of Apatinib or a pharmaceutically acceptable salt thereof in combination with an anti-PD-1 antibody or antigen-binding fragment thereof in the manufacture of a medicament for the treatment of Triple Negative Breast Cancer.

The present invention also provides use of an anti-PD-1 antibody or antigen-binding fragment thereof in the manufacture of a medicament for the treatment of Triple Negative Breast Cancer.

As used herein, Triple Negative Breast Cancer refers to breast cancer wherein the estrogen receptor (ER), the progesterone receptor (PR) and the proto-oncogene Her-2 are all negative.

In a preferred embodiment of the invention, the anti-PD-1 antibody or antigen-binding fragment thereof is selected from the group consisting of AMP-224, GLS-010, IBI-308, REGN-2810, PDR-001, BGB-A317, Pidilizumab, PF-06801591, Genolimzumab, CA-170, MEDI-0680, JS-001, TSR-042, Camrelizumab, Pembrolizumab, LZM-009, AK-103 and Nivolumab.

In a preferred embodiment of the present invention, the light chain variable region of the anti-PD-1 antibody or antigen-binding fragment thereof comprises LCDR1, LCDR2 and LCDR3 as shown in SEQ ID NO:4, SEQ ID NO:5 and SEQ ID NO: 6, respectively; and the heavy chain variable region comprises HCDR1, HCDR2 and HCDR3 as shown in SEQ ID NO:1, SEQ ID NO:2, and SEQ ID NO:3, respectively.

Each CDR sequence described above is shown in the following table:

| Name | Sequence | SEQID NO |
|---|---|---|
| HCDR1 | SYMMS | SEQID NO: 1 |
| HCDR2 | TISGGGANTYYPDSVKG | SEQID NO: 2 |
| HCDR3 | QLYYFDY | SEQID NO: 3 |
| LCDR1 | LASQTIGTWLT | SEQID NO: 4 |
| LCDR2 | TATSLAD | SEQID NO: 5 |
| LCDR3 | QQVYSIPWT | SEQID NO: 6 |

Preferably, the PD-1 antibody is a humanized antibody.

Preferably, the humanized antibody light chain variable region sequence is shown in SEQ ID NO:10 or a variant thereof; preferably, said variant has 0-10 amino acid changes in the light chain variable region; More preferably, the amino acid change is A43 S. The humanized antibody heavy chain variable region sequence is shown in SEQ ID NO:9 or a variant thereof; preferably, said variant has 0-10 amino acid change in the heavy chain variable region; More preferably, the amino acid change is G44R.

The humanized antibody heavy and light chain variable region sequences are as follows:
Heavy chain variable region 
EVQLVESGGGLVQPGGSLRLSCAASGFTFSSYMMSWVRQAPGKGLEWVATISG
GGANTYYPDSVKGRFTISRDNAKNSLYLQMNSLRAEDTAVYYCARQLYYFDYW
GQGTTVTVSS SEQID NO: 9
Light chain variable region

DIQMTQSPSSLSASVGDRVTITCLASQTIGTWLTWYQQKPGKAPKLLIYTATSLA
DGVPSRFSGSGSGTDFTLTISSLQPEDFATYYCQQVYSIPWTFGGGTKVEIK
SEQID NO: 10

A preferred humanized antibody light chain sequence is shown in SEQ ID NO:8 or a variant thereof; Preferably, said variant has 0-10 amino acid changes in the light chain variable region; More preferably, the amino acid change is A43S. The humanized antibody heavy chain sequence is shown in SEQ ID NO:7 or a variant thereof; Preferably, said variant has 0-10 amino acid changes in the heavy chain variable region; More preferably, the amino acid change is G44R.

In a preferred embodiment of the invention, the humanized antibody light chain sequence is shown in SEQ ID NO:8, and the heavy chain sequence is shown in SEQ ID NO:7.

The sequences of the humanized antibody heavy and light chain are as follows:
Heavy chain 
EVQLVESGGGLVQPGGSLRLSCAASGFTFSSYMMSWVRQAPGKGLEWVATISG
GGANTYYPDSVKGRFTISRDNAKNSLYLQMNSLRAEDTAVYYCARQLYYFDYW
GQGTTVTVSSASTKGPSVFPLAPCSRSTSESTAALGCLVKDYFPEPVTVSWNSGA
 
LTSGVHTFPAVLQSSGLYSLSSVVTVPSSSLGTKTYTCNVDHKPSNTKVDKRVES
KYGPPCPPCPAPEFLGGPSVFLFPPKPKDTLMISRTPEVTCVVVDVSQEDPEVQF
NWYVDGVEVHNAKTKPREEQFNSTYRVVSVLTVLHQDWLNGKEYKCKVSNK
GLPSSIEKTISKAKGQPREPQVYTLPPSQEEMTKNQVSLTCLVKGFYPSDIAVEWE
SNGQPENNYKTTPPVLDSDGSFFLYSRLTVDKSRWQEGNVFSCSVMHEALHNH
YTQKSLSLSLGK
SEQID NO: 7
Light chain

DIQMTQSPSSLSASVGDRVTITCLASQTIGTWLTWYQQKPGKAPKLLIYTATSLA
DGVPSRFSGSGSGTDFTLTISSLQPEDFATYYCQQVYSIPWTFGGGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
SEQID NO: 8

In a preferred embodiment of the present invention, the Triple Negative Breast Cancer patient is the one who failed in chemotherapy or intolerant to chemotherapy.

In a preferred embodiment of the invention, the chemotherapeutic agent is one or more selected from the group consisting of anthracyclines, taxoids, vinorelbine, capecitabine, gemcitabine and platinum-based drugs.

The anthracyclines of the present invention include doxorubicin, epirubicin, idarubicin, daunorubicin, nemorubicin and derivatives thereof, and the like.

The taxoids of the present invention include paclitaxel, docetaxel, paclitaxel liposomes, albumin-bound paclitaxel, and the like.

The platinum-based drugs of the present invention include carboplatin, cisplatin, oxaliplatin, Nedaplatin, lobaplatin, satraplatin, cycloplatin, Miboplatin, Enloplatin, Iproplatin, Dicycloplatin, and the like.

In a preferred embodiment of the invention, the dose of the PD-1 antibody or antigen-binding fragment thereof is from 1 to 10mg/kg, preferably is 1 mg/kg, 2 mg/kg, 3 mg/kg, 4 mg/kg, 5 mg/kg, 6mg/kg, 7mg/kg, 8mg/kg, 9mg/kg or 10 mg/kg, more preferably is 1 mg/kg, 3 mg/kg, 4 mg/kg, 5 mg/kg, 6mg/kg or 10 mg/kg.

In a preferred embodiment of the present invention, wherein the dose of the PD-1 antibody or antigen-binding fragment thereof is from 50 to 600mg, preferably is 50 mg, 60mg, 70mg, 75mg, 100 mg, 125mg, 150 mg, 175mg, 200mg, 225mg, 250mg, 375mg, 400 mg, 425mg, 450mg, 475mg, 500 mg or 600mg, more preferably is 60mg, 100 mg, 200 mg, 400 mg or 600mg.

In a preferred embodiment of the invention, wherein the dose of Apatinib or a pharmaceutically acceptable salt thereof is from 100 to 500mg, preferably is 100 mg, 125mg, 150 mg, 175mg, 200 mg, 225mg, 250mg, 275mg, 300 mg, 325 mg, 350 mg, 375 mg, 400 mg or 500 mg, more preferably is 200 mg, 225mg, 250mg, 275mg, 300 mg, 325 mg, 350 mg or 375 mg.

In a preferred embodiment of the present invention, the PD-1 antibody or antigen-binding fragment thereof is administered at a frequency of once a week, once every two week, once every three week, or once a month, and Apatinib or a pharmaceutically acceptable salt thereof is administered at a frequency of once a day, once every two day, once every three day, 5 days of administration and 2 days of withdrawal, or 7 days of administration and 7 days of withdrawal.

As used herein, "in combination with" refers to mode of administration, particularly, it refers to administration of at least one dose of Apatinib and at least one dose of PD-1 antibody over a period of time, wherein both substances exhibit pharmacological effects. The period of time may be within one administration cycle, preferably within 4 weeks, within 3 weeks, within 2 weeks, within 1 week, or within 24 hours, more preferably within 12 hours. Apatinib and the PD-1 antibody may be administered simultaneously or sequentially. The following treatment is encompassed in such periods: Apatinib and the PD-1 antibody are administered via the same route of administration or via different routes of administration. The mode of co-administration described herein is selected from the group consisting of simultaneous administration, co-administration of independent formulations and sequential administration of independent formulations. The route of co-administration described herein is selected from the group consisting of oral administration, parenteral administration and transdermal administration, the parenteral administration includes, but not limited to, intravenous injection, subcutaneous injection, and intramuscular injection.

In a preferred embodiment of the present invention, at the time of administration, the PD-1 antibody or antigen-binding fragment thereof is administered at an amount of 60 to 600mg, by intravenous infusion, once every one to three weeks; Apatinib or a pharmaceutically acceptable salt thereof is administered at an amount of 250mg to 500 mg, orally, once every one to two days.

In a preferred embodiment of the present invention, at the time of administration, the PD-1 antibody or antigen-binding fragment thereof is administered at an amount of 60 to 600mg, by intravenous infusion, once every one to three weeks; Apatinib or a pharmaceutically acceptable salt thereof is administered at an amount of 250mg to 500 mg, orally, 5 days of administration and 2 days of withdrawal .

In a preferred embodiment of the present invention, at the time of administration, the PD-1 antibody or antigen-binding fragment thereof is administered at an amount of 60 to 600mg, by intravenous infusion, once every one to three weeks; Apatinib or a pharmaceutically acceptable salt thereof is administered at an amount of 250mg to 500 mg, orally, 7 days of administration and 7 days of withdrawal.

In a preferred embodiment of the present invention, at the time of administration, the PD-1 antibody or antigen-binding fragment thereof is administered at an amount of 200mg, by intravenous infusion, once every two weeks; Apatinib or a pharmaceutically acceptable salt thereof is administered at an amount of 375 mg, orally, once a day.

In a preferred embodiment of the present invention, at the time of administration, the PD-1 antibody or antigen-binding fragment thereof is administered at an amount of 200mg, by intravenous infusion, once every two weeks; Apatinib or a pharmaceutically acceptable salt thereof is administered at an amount of 375 mg, orally, 5 days of administration and 2 days of withdrawal.

In a preferred embodiment of the present invention, at the time of administration, the PD-1 antibody or antigen-binding fragment thereof is administered at an amount of 200mg, by intravenous infusion, once every two weeks; Apatinib or a pharmaceutically acceptable salt thereof is administered at an amount of 375 mg, orally, 7 days of administration and 7 days of withdrawal.

In a preferred embodiment of the present invention, at the time of administration, the PD-1 antibody or antigen-binding fragment thereof is administered at an amount of 200mg, by intravenous infusion, once every two weeks; Apatinib or a pharmaceutically acceptable salt thereof is administered at an amount of 250 mg, orally, once a day.

In a preferred embodiment of the present invention, at the time of administration, the PD-1 antibody or antigen-binding fragment thereof is administered at an amount of 200mg, by intravenous infusion, once every two weeks; Apatinib or a pharmaceutically acceptable salt thereof is administered at an amount of 250 mg, orally, 5 days of administration and 2 days of withdrawal.

In a preferred embodiment of the present invention, at the time of administration, the PD-1 antibody or antigen-binding fragment thereof is administered at an amount of 200mg, by intravenous infusion, once every two weeks; Apatinib or a pharmaceutically acceptable salt thereof is administered at an amount of 250 mg, orally, 7 days of administration and 7 days of withdrawal.

In a preferred embodiment of the invention, the PD-1 antibody is administered by injection, for example subcutaneous or intravenous injection, and the PD-1 antibody should be formulated as an injectable form prior to injection. A particularly preferred injectable form of the PD-1 antibody is injection solution or lyophilized powder for injection, comprising the PD-1 antibody, buffer, stabilizer, and optionally surfactant. The buffer may be one or more selected from the group consisting of acetate, citrate, succinate and phosphate. The stabilizer may be saccharides or amino acids, preferably disaccharides, such as sucrose, lactose, trehalose, maltose. The surfactant is selected from the group consisting of polyoxyethylene hydrogenated castor oil, glycerol fatty acid esters and polyoxyethylene sorbitan fatty acid esters, preferably the polyoxyethylene sorbitan fatty acid esters are polysorbate 20, 40, 60 or 80, most preferably polysorbate 20. Most preferably, the injectable form of PD-1 antibody includes a PD-1 antibody, acetate buffer, trehalose, and polysorbate 20.

The present invention provides the anti-PD-1 antibody or an antigen-binding fragment thereof as described above in combination with Apatinib or a pharmaceutically acceptable salt thereof as a medicament for reducing adverse effects of the medicament, preferably, the adverse effects of the medicament are caused by the anti-PD-1 antibody or antigen-binding fragment thereof or Apatinib or a pharmaceutically acceptable salt thereof.

In a preferred embodiment of the present invention, the adverse effects of the medicament mediated by the anti-PD-1 antibody or antigen-binding fragment thereof and/or immunity can be reduced, when the PD-1 antibody or antigen-binding fragment thereof is used in combination with Apatinib or a pharmaceutically acceptable salt thereof; Preferably, the adverse effect is vascular-related adverse effect.

The present invention also provides a pharmaceutical kit or pharmaceutical package, comprising the above anti-PD-1 antibody or antigen-binding fragment thereof and the above Apatinib or a pharmaceutically acceptable salt thereof.

The present invention also provides a method of treating Triple Negative Breast Cancer, comprising administering to a patient the above PD-1 antibody or antigen-binding fragment thereof and the above Apatinib or a pharmaceutically acceptable salt thereof.

The present invention also provides a pharmaceutical composition, comprising an effective amount of an PD-1 antibody or antigen-binding fragment thereof and Apatinib or a pharmaceutically acceptable salt thereof, as described above, and one or more pharmaceutically acceptable excipients, diluents or carriers.

### Detailed description

### I. Terminology

In order to make the invention more readily be understood, certain technical and scientific terms are specifically defined below. All other technical and scientific terms used herein have the meanings commonly understood by one of ordinary skill in the art to which this invention belongs, unless it is obvious that they have clear definitions in this document.

The term "humanized antibody", also known as CDR-grafted antibody (CDR-grafted antibody), refers to an antibody produced by grafting the mouse CDR sequences into the human antibody variable region frameworks, i.e., produced in different types of human germline antibody framework sequences. Such humanized antibodies can overcome the strong antibody responses induced by large amounts of mouse protein components carried by chimeric antibodies. Such framework sequences may be obtained from public DNA databases or published references including germline antibody genetic sequences. For example, human heavy and light chain variable region germline DNA sequences can be found in "VBase", a human germline sequence database, (available on the Internet www.mrccpe.com.ac.uk/vbase), and in Kabat, E.A. et al., 1991 Sequences of Proteins of Immunological Interest, 5th edition. In a preferred embodiment of the invention, the CDR sequences of the humanized PD-1 antibody is selected from the group consisting of SEQ ID NO:1, 2, 3, 4, 5 and 6.

The term "antigen-binding fragment", refers to Fab fragment, Fab' fragment, and F(ab')2 fragment having antigen-binding activity, as well as Fv fragment and sFv fragment that binds to human PD-1. The "antigen-binding fragment" comprises one or more CDR regions selected from SEQ ID NO:1 to SEQ ID NO:6 of the antibody of the invention. The Fv fragment contains an antibody heavy chain variable region and a light chain variable region, without constant region, and is a minimal antibody fragment with all of the antigen binding sites. Generally, the Fv antibody also comprises a polypeptide linker between the VH and VL domains, capable of forming the structure required for antigen binding. The two antibody variable regions may also be connected by different linkers to form a polypeptide chain called as single chain antibody or single chain Fv (sFv). The term "binding to PD-1" as used in the present invention means being capable of interacting with human PD-1. The term "antigen binding sites" as used in the present invention refers to discrete three-dimensional sites on an antigen recognized by the antibody or antigen binding fragment of the present invention.

As used herein, the term "failed in treatment" means that the subject has a measurable tumor lesion at the baseline, and is classified as a progressive disease (PD) or intolerant according to the RECIST 1.1 Efficacy Evaluation Criteria.

"Intolerant" as used herein means that the treatment cannot be continued due to adverse effects caused by the medicament.

Overall survival (OS) refers to the period from a random period to the death due to any cause. For the subjects who were still alive at the last follow-up, OS was recorded as censored data at time of the last follow-up. For the subjects who lost follow-up, OS was recorded as censored data at the time of last confirmation of survival, before the lost of follow-up. OS with censored data is defined as the period from random grouping to censoring data.

Objective response rate (ORR) refers to the proportion of patients whose tumors are reduced to a certain extent and maintain the level for a certain period of time, including cases of CR and PR. Objective response of tumor is assessed according to the tumor response assessment criteria (RECIST 1.1 criteria). Subjects must have measurable tumor lesions at baseline, and the efficacy was classified as complete response (CR), partial response (PR), stable disease (SD), and progressive disease (PD) according to RECIST 1.1 criteria.

Disease Control Rate (DCR) refers to the percentage of patients who have been confirmed as complete response, partial response, and stable disease (≥ 8 weeks) in patients to whom the efficacy is evaluable.

Complete response (CR): All target lesions disappear and all pathological lymph nodes (including target and non-target nodules) have less than 10 mm short diameter.

Partial response (PR): The sum of diameters of target lesions is reduced by at least 30% compared to the baseline level.

Progressive disease (PD): The minimum value of the sum of diameters of all measured target lesions during the experimental study is used as a reference, the sum of diameters is relatively increased by at least 20% (the value measured at baseline will be used as a reference, if it is minimum); In addition, the absolute value of the sum of the diameters must be increased by at least 5 mm (development of one or more new lesions is also considered as progressive disease).

Stable disease (SD): The extent of reduction of target lesions does not satisfy PR and the extent of increase does not satisfy PD. SD falls in between PR and PD. The minimum value of the sum of diameters can be used as a reference.

### DESCRIPTION OF THE DRAWINGS

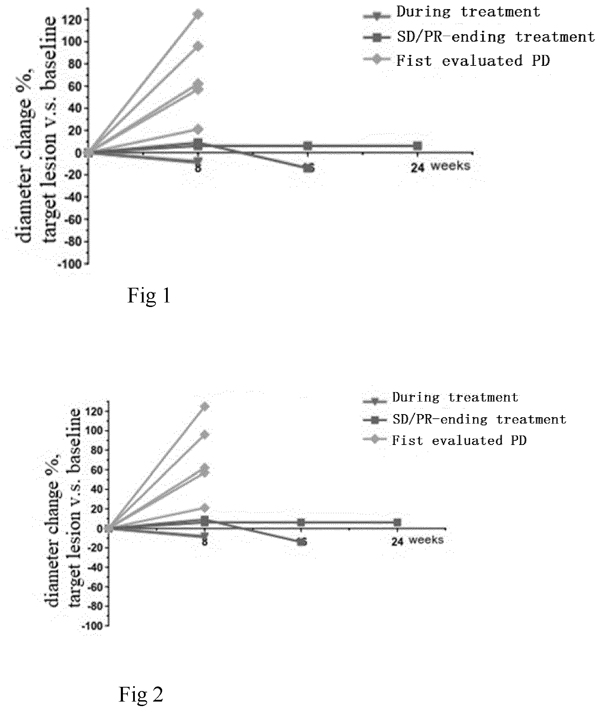

Figure 1 shows the changes in diameter values of the target lesions between the continuous Apatinib administration group and the baseline.
Figure 2 shows the changes in diameter values of the target lesions between the Apatinib group (7 days of administration and 7 days of withdrawal) and the baseline.

### DETAILED DESCRIPTION OF THE DISCLOSURE

The invention is further described below in connection with examples, but these examples are not intended to limit the scope of the invention.

### Example 1: Clinical study of anti-PD-1 antibody in combination with Apatinib mesylate for the treatment of Triple Negative Breast Cancer

### 1. Antibodies and Compounds to be tested

The PD-1 antibody has a heavy chain and a light chain as shown in SEQ ID NO:7 and SEQ ID NO:8 in the present invention. 20mg/ml PD-1 antibody was prepared from 200mg/vial for use.

Apatinib mesylate tablets are commercially available.

2. Enrollment criteria: (1) Recurrent and metastatic Triple Negative Breast Cancer was pathologically confirmed (ER negative (IHC ER positive percentage <1%), PR negative (IHC PR positive percentage <1%), HER2 negative (IHC-/+ or IHC++ but FISH/CISH-)); (2) The patients have been treated with anthracyclines and taxoids, but failed to the treatment. In the phase of recurrence and metastasis, the chemotherapeutic lines used <3 lines; (3) The patients have measurable lesions; (4) ECOG score is 0-1.

### 3. Exemplary dosing regimens of PD-1 and Apatinib are as follows:

200 mg PD-1 antibody, intravenously infused, once every two weeks + 375mg Apatinib, orally, once a day;
200 mg PD-1 antibody, intravenously infused, once every two weeks + 375mg Apatinib, orally, 7 days of administration and 7 days of withdrawal;
200 mg PD-1 antibody, intravenously infused, once every two weeks + 250 mg Apatinib, orally, once a day;
200 mg PD-1 antibody, intravenously infused, once every two weeks + 250 mg Apatinib, orally, 7 days of administration and 7 days of withdrawal.

### 4. Dosing Regimen

The PD-1 antibody was administered intravenously at a fixed dose of 200 mg, via intravenous drip for 30 min (not less than 20 min, no more than 60 min), once every 2 weeks, in a cycle of 4 weeks, with a maximum administration duration of 2 years.

Apatinib was administered orally after meals, once a day, one tablet each time, (250mg/tablet or 375mg/tablet), 7 days of administration, 7 or 14 days of withdrawal.

### 5. Dosage adjustment

Based on the toxic and adverse effects of the study drug, the dosing of the study drugs may be paused, downregulated and terminated during the study.

The administration of the PD-1 antibody was allowed to be paused during the study, for up to 8 weeks; when the administration of the PD-1 antibody was delayed for more than 3 days, no administration was given for the current point, and a dose of 200 mg would be administered until the next time of administration as scheduled.

In addition to the pause of dosing, the dose of the PD-1 antibody may be downregulated to 3 mg/kg, q2w, for underweighted subjects.

Due to Apatinib-related toxicity, dosage adjustments included: pause of dosing (no more than 28 days), down-regulation of dose (375mg/d dose group), and termination of dosing. It was only allowed to downregulate the dose of Apatinib during the study, and the dose of Apatinib may be down-regulated from 375mg/d to 250mg/d. However, it was not allowed to upregulate the dose of Apatinib. When Apatinib-related toxicities were observed, the administration should be paused first, and then administered with the original dose, downregulated dose or terminated administration, dependent on the recovery from the toxicity. After the administration of Apatinib was terminated, the administration of the PD-1 antibody monotherapy to the subject may be continued.

### 6. Results

Two groups of patients received with 200 mg PD-1 antibody, IV, q2W; 250mg Apatinib daily oral; and PD-1 antibody, IV, q2W; 250mg of Apatinib administered in a cycle of 14 days (including 7 days of administration, 7 days of withdrawal); respectively. 19 patients were enrolled, 10 patients were received with continuous administration, and 9 patients were administered for 7 days with 7 days of withdrawal. Seventeen patients were evaluable, wherein eight patients were in the continuous administration group and nine patients were in the group of 7 days-administration plus 7 days-withdrawal. The first evaluation of efficiency was performed in the continuous administration group, wherein PR: 3 patients, SD: 2 patients, PD 3 patients, ORR was 37.5%, DCR was 62.5%; As for the group of 7 days-administration plus 7 days-withdrawal, wherein SD: 4 patients, PD: 5 patients, ORR was 0%, DCR was 44.4%.

Some subjects were subsequently enrolled, to a total of 23 patients, with the following results:

| | Number of patients | Evaluable | PR | Confirmed PR | SD | ORR | DCR |
|---|---|---|---|---|---|---|---|
| Intermittent administration group | 10 | 9 | 0 | 0 | 4 | 0 | 44.40% |
| Continuous administration group | 13 | 13 | 6 | 5 | 3 | 38.50% | 61.50% |

Co-administration of the PD-1 antibody and Apatinib in present invention was tolerable and safe in subjects with Triple Negative Breast Cancer. Most subjects terminated the treatment due to the progressive disease. Substantially no capillary hemangioma was observed in the continuous administration group, significantly superior to the incidence of capillary hemangioma occurred in PD-1 antibody monotherapy used in phase I clinical trial.

## Claims

1. Use of an anti-PD-1 antibody or antigen-binding fragment thereof in combination with Apatinib or a pharmaceutically acceptable salt thereof in the manufacture of a medicament for the treatment of Triple Negative Breast Cancer.

2. The use of claim 1, wherein the anti-PD-1 antibody or antigen-binding fragment thereof is selected from the group consisting of AMP-224, GLS-010, IBI-308, REGN-2810, PDR-001, BGB-A317, Pidilizumab, PF-06801591, Genolimzumab, CA-170, MEDI-0680, JS-001, TSR-042, Camrelizumab, Pembrolizumab, LZM-009, AK-103 and Nivolumab.

3. The use of claim 1, wherein the light chain variable region of the anti-PD-1 antibody or antigen-binding fragment thereof comprises LCDR1, LCDR2 and LCDR3 as shown in SEQ ID NO:4, SEQ ID NO:5 and SEQ ID NO: 6, respectively, and the heavy chain variable region of the anti-PD-1 antibody comprises HCDR1, HCDR2 and HCDR3 as shown in SEQ ID NO:1, SEQ ID NO:2 and SEQ ID NO:3, respectively.

4. The use of claim 3, wherein the anti-PD-1 antibody is a humanized antibody.

5. The use of claim 4, wherein the light chain variable region sequence of humanized antibody is shown in SEQ ID NO:10 or a variant thereof; preferably, said variant has 0-10 amino acid changes in the light chain variable region; more preferably, the amino acid change is A43S; the heavy chain variable region sequence is shown in SEQ ID NO:9 or a variant thereof; preferably, said variant has 0-10 amino acid change in the heavy chain variable region; more preferably, the amino acid change is G44R.

6. The use of claim 5, wherein the light chain sequence of humanized antibody is shown in SEQ ID NO:8 or a variant thereof; preferably, said variant has 0-10 amino acid changes in the light chain variable region; more preferably, the amino acid change is A43S; the heavy chain sequence is shown in SEQ ID NO:7 or a variant thereof; preferably, said variant has 0-10 amino acid changes in the heavy chain variable region; more preferably, the amino acid change is G44R.

7. The use of claim 6, wherein the light chain sequence of humanized antibody is shown in SEQ ID NO:8 and the heavy chain sequence is shown in SEQ ID NO:7.

8. The use of any one of claims 1-7, wherein the chemotherapy has failed in the treatment of the Triple Negative Breast Cancer.

9. The use of claim 8, wherein the chemotherapeutic agent is one or more selected from the group consisting of anthracyclines, taxoids, vinorelbine, capecitabine, gemcitabine and platinum-based drugs.

10. The use of any one of claims 1 to 9, wherein the dose of the PD-1 antibody or antigen-binding fragment thereof is from 1 to 10mg/kg, preferably is 1 mg/kg, 2 mg/kg, 3 mg/kg, 4 mg/kg, 5 mg/kg, 6mg/kg, 7mg/kg, 8mg/kg, 9mg/kg or 10 mg/kg, more preferably is 1 mg/kg, 3 mg/kg, 4 mg/kg, 5 mg/kg, 6mg/kg or 10 mg/kg.

11. The use of any one of claims 1 to 9, wherein the dose of the PD-1 antibody or antigen-binding fragment thereof is from 50 to 600mg, preferably is 50 mg, 60mg, 70mg, 75mg, 100 mg, 125mg, 150 mg, 175mg, 200mg, 225mg, 250mg, 375mg, 400 mg, 425mg, 450mg, 475mg, 500 mg or 600mg, more preferably is 60mg, 100 mg, 200 mg, 400 mg or 600mg.

12. The use of any one of claims 1 to 9, wherein the dose of Apatinib or a pharmaceutically acceptable salt thereof is from 100 to 500mg, preferably is 100 mg, 125mg, 150 mg, 175mg, 200 mg, 225mg, 250mg, 275mg, 300 mg, 325 mg, 350 mg, 375 mg, 400 mg or 500 mg, more preferably is 200 mg, 225mg, 250mg, 275mg, 300 mg, 325 mg, 350 mg or 375 mg.

13. A pharmaceutical package, comprising Apatinib or a pharmaceutically acceptable salt thereof and an anti-PD-1 antibody or antigen-binding fragment thereof according to any one of claims 1 to 12.

14. A pharmaceutical composition, comprising an effective amount of an PD-1 antibody or antigen-binding fragment thereof and Apatinib or a pharmaceutically acceptable salt thereof according to any one of claims 1 to 12, and one or more pharmaceutically acceptable excipients, diluents or carriers.


Sequence listing

<110>  JIANGSU HENGRUI MEDICINE CO., LTD.;
       SUZHOU SUNCADIA BIOPHARMACEUTICALS CO., LTD.

<120>  USE OF COMBINED TREATMENT OF PD-1 ANTIBODY AND APATINIB FOR TREATING
TRIPLE NEGATIVE BREAST CANCER

<160>  10

<170>  SIPOSequenceListing 1.0

<210>  1
<211>  5
<212>  PRT
<213>  Mus musculus

<400>  1
Ser Tyr Met Met Ser
1               5

<210>  2
<211>  17
<212>  PRT
<213>  Mus musculus

<400>  2
Thr Ile Ser Gly Gly Gly Ala Asn Thr Tyr Tyr Pro Asp Ser Val Lys
1               5                   10                  15
Gly

<210>  3
<211>  7
<212>  PRT
<213>  Mus musculus

<400>  3
Gln Leu Tyr Tyr Phe Asp Tyr
1               5

<210>  4
<211>  11
<212>  PRT
<213>  Mus musculus

<400>  4
Leu Ala Ser Gln Thr Ile Gly Thr Trp Leu Thr
1               5                   10

<210>  5
<211>  7
<212>  PRT
<213>  Mus musculus

<400>  5
Thr Ala Thr Ser Leu Ala Asp
1               5

<210>  6
<211>  9
<212>  PRT
<213>  Mus musculus

<400>  6
Gln Gln Val Tyr Ser Ile Pro Trp Thr
1               5

<210>  7
<211>  443
<212>  PRT
<213>  Artificial Sequence

<220>
<221>  PEPTIDE
<222>  (1)..(443)
<223>  heavy chain sequence


<400>  7
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Met Met Ser Trp Val Arg Gln Ala Pro Gly Lys Gly Leu Glu Trp Val
        35                  40                  45
Ala Thr Ile Ser Gly Gly Gly Ala Asn Thr Tyr Tyr Pro Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Gln Leu Tyr Tyr Phe Asp Tyr Trp Gly Gln Gly Thr Thr Val
            100                 105                 110
Thr Val Ser Ser Ala Ser Thr Lys Gly Pro Ser Val Phe Pro Leu Ala
        115                 120                 125
Pro Cys Ser Arg Ser Thr Ser Glu Ser Thr Ala Ala Leu Gly Cys Leu
    130                 135                 140
Val Lys Asp Tyr Phe Pro Glu Pro Val Thr Val Ser Trp Asn Ser Gly
145                 150                 155                 160
Ala Leu Thr Ser Gly Val His Thr Phe Pro Ala Val Leu Gln Ser Ser
                165                 170                 175
Gly Leu Tyr Ser Leu Ser Ser Val Val Thr Val Pro Ser Ser Ser Leu
            180                 185                 190
Gly Thr Lys Thr Tyr Thr Cys Asn Val Asp His Lys Pro Ser Asn Thr
        195                 200                 205
Lys Val Asp Lys Arg Val Glu Ser Lys Tyr Gly Pro Pro Cys Pro Pro
    210                 215                 220
Cys Pro Ala Pro Glu Phe Leu Gly Gly Pro Ser Val Phe Leu Phe Pro
225                 230                 235                 240
Pro Lys Pro Lys Asp Thr Leu Met Ile Ser Arg Thr Pro Glu Val Thr
                245                 250                 255
Cys Val Val Val Asp Val Ser Gln Glu Asp Pro Glu Val Gln Phe Asn
            260                 265                 270
Trp Tyr Val Asp Gly Val Glu Val His Asn Ala Lys Thr Lys Pro Arg
        275                 280                 285
Glu Glu Gln Phe Asn Ser Thr Tyr Arg Val Val Ser Val Leu Thr Val
    290                 295                 300
Leu His Gln Asp Trp Leu Asn Gly Lys Glu Tyr Lys Cys Lys Val Ser
305                 310                 315                 320
Asn Lys Gly Leu Pro Ser Ser Ile Glu Lys Thr Ile Ser Lys Ala Lys
                325                 330                 335
Gly Gln Pro Arg Glu Pro Gln Val Tyr Thr Leu Pro Pro Ser Gln Glu
            340                 345                 350
Glu Met Thr Lys Asn Gln Val Ser Leu Thr Cys Leu Val Lys Gly Phe
        355                 360                 365

Tyr Pro Ser Asp Ile Ala Val Glu Trp Glu Ser Asn Gly Gln Pro Glu
    370                 375                 380
Asn Asn Tyr Lys Thr Thr Pro Pro Val Leu Asp Ser Asp Gly Ser Phe
385                 390                 395                 400
Phe Leu Tyr Ser Arg Leu Thr Val Asp Lys Ser Arg Trp Gln Glu Gly
                405                 410                 415
Asn Val Phe Ser Cys Ser Val Met His Glu Ala Leu His Asn His Tyr
            420                 425                 430
Thr Gln Lys Ser Leu Ser Leu Ser Leu Gly Lys
        435                 440

<210>  8
<211>  214
<212>  PRT
<213>  Artificial Sequence

<220>
<221>  PEPTIDE
<222>  (1)..(214)
<223>  light chain sequence


<400>  8
Asp Ile Gln Met Thr Gln Ser Pro Ser Ser Leu Ser Ala Ser Val Gly
1               5                   10                  15
Asp Arg Val Thr Ile Thr Cys Leu Ala Ser Gln Thr Ile Gly Thr Trp
            20                  25                  30
Leu Thr Trp Tyr Gln Gln Lys Pro Gly Lys Ala Pro Lys Leu Leu Ile
        35                  40                  45
Tyr Thr Ala Thr Ser Leu Ala Asp Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Val Tyr Ser Ile Pro Trp
                85                  90                  95
Thr Phe Gly Gly Gly Thr Lys Val Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210

<210>  9
<211>  116
<212>  PRT
<213>  Artificial Sequence

<220>
<221>  PEPTIDE
<222>  (1)..(116)
<223>  heavy chain variable region

<400>  9
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Met Met Ser Trp Val Arg Gln Ala Pro Gly Lys Gly Leu Glu Trp Val
        35                  40                  45
Ala Thr Ile Ser Gly Gly Gly Ala Asn Thr Tyr Tyr Pro Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Ala Arg Gln Leu Tyr Tyr Phe Asp Tyr Trp Gly Gln Gly Thr Thr Val
            100                 105                 110
Thr Val Ser Ser
        115

<210>  10
<211>  107
<212>  PRT
<213>  Artificial Sequence

<220>
<221>  PEPTIDE
<222>  (1)..(10)
<223>  light chain variable region


<400>  10
Asp Ile Gln Met Thr Gln Ser Pro Ser Ser Leu Ser Ala Ser Val Gly
1               5                   10                  15
Asp Arg Val Thr Ile Thr Cys Leu Ala Ser Gln Thr Ile Gly Thr Trp
            20                  25                  30
Leu Thr Trp Tyr Gln Gln Lys Pro Gly Lys Ala Pro Lys Leu Leu Ile
        35                  40                  45
Tyr Thr Ala Thr Ser Leu Ala Asp Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Val Tyr Ser Ile Pro Trp
                85                  90                  95
Thr Phe Gly Gly Gly Thr Lys Val Glu Ile Lys
            100                 105